# EUROPEAN PATENT SPECIFICATION

(11) **EP 2 979 696 B1**
(45) Date of publication and mention of the grant of the patent: **08.05.2019**
(21) Application number: 14772608.7
(22) Date of filing: 27.03.2014
(51) Int. Cl.: A61K 31/40, A61K 31/404, A61K 31/4035, A61P 35/00, A61K 31/426

(54) **PHARMACEUTICAL COMPOSITION FOR PREVENTING OR TREATING OVARY GRANULOSA CELL TUMORS CONTAINING GLYCOGEN SYNTHASE KINASE-3 BETA INHIBITOR AS ACTIVE INGREDIENT, AND FUNCTIONAL HEALTH FOOD COMPOSITION**
PHARMAZEUTISCHE ZUSAMMENSETZUNG ZUR PRÄVENTION ODER BEHANDLUNG VON EIERSTOCK-GRANULOSAZELLTUMOREN MIT EINEM GLYCOGEN-SYNTHASE-KINASE-3-BETA-HEMMER ALS WIRKSTOFF UND FUNKTIONELLE GESUNDHEITSNAHRUNGSMITTELZUSAMMENSETZUNG
COMPOSITION PHARMACEUTIQUE POUR PRÉVENIR OU TRAITER LES TUMEURS DE LA GRANULOSA DE L'OVAIRE CONTENANT UN INHIBITEUR DE GLYCOGÉNÉSYNTHÉTASE KINASE-3 BETA COMME PRINCIPE ACTIF, ET UNE COMPOSITION FONCTIONNELLE D'ALIMENTS NATURELS

(30) Priority: 29.03.2013 KR 20130034672
(43) Date of publication of application: 03.02.2016
(73) Proprietor: Chung-Ang University Industry-Academic Cooperation Foundation, Seoul 156-756 (KR)
(72) Inventor: BAE, Jee Hyeon, Gwangju-si Gyeonggi-do 464-874 (KR); LEE, Kang Seok, Gwangju-si Gyeonggi-do 464-874 (KR); KIM, Jaehong, Busan 608-842 (KR)
(74) Representative: Dehns
(86) International application number: PCT/KR2014/002619
(87) International publication number: WO 2014/157966

(56) References cited:
- WO-A1-2010/040204
- WO-A2-2006/034207
- WO-A2-2006/063164
- WO-A2-2012/135588
- CN-A- 102 258 783
- US-A1- 2002 052 397
- US-A1- 2013 071 495
- M.-N. LAGUE ET AL: "Synergistic effects of Pten loss and WNT/CTNNB1 signaling pathway activation in ovarian granulosa cell tumor development and progression", CARCINOGENESIS., vol. 29, no. 11, 6 August 2008 (2008-08-06), pages 2062-2072, XP055322850, GB ISSN: 0143-3334, DOI: 10.1093/carcin/bgn186
- S. UZBEKOVA ET AL.: 'Glycogen synthase kinase 3B in bovine oocytes and granulosa cells: possible involvement in meiosis during in vitro maturation' REPRODUCTION vol. 138, 2009, pages 235 - 246, XP055285131

## Description

### [Technical Field]

The present invention relates to a pharmaceutical composition and functional health food composition for preventing or treating a granulosa cell tumor of the ovary, and more particularly, to a pharmaceutical composition and functional health food composition for preventing or treating a granulosa cell tumor of the ovary containing a glycogen synthase kinase-3 beta inhibitor as an active ingredient.

### [Background Art]

A granulosa cell tumor (GCT) of the ovary and a theca cell tumor of the ovary are the predominant types of the diseases easily occurring in women in their twenties. Among these, the GCT of the ovary is the most common type of a malignant sexcord-stromal tumor (SCST), produces estrogen and thus has a symptom of irregular menstruation before premenarcheal vaginal bleeding or vaginal bleeding after menopause. 3/4 of GCTs correspond to the above type of a tumor, the tumors are characterized by a low growth rate and a very long relapse time, and thus a disease-free survival time is over 10 years.

However, for treatment, a surgical treatment such as a hysterectomy or laparosalpingectomy, radiotherapy or chemotherapy may be used, the surgical treatment has side effects such as bleeding, infection, etc. or postoperative sequela, the radiotherapy has serious side effects caused by radiation exposure, and an effect of the chemotherapy has not been proved. The GCT of the ovary comprises 5% or less of the total ovary cancers, but 90% or more of the patients diagnosed and treated before their twenties can be cured at an early stage, and therefore the development of a composition effective for preventing, improving or treating a GCT of the ovary is very important.

Meanwhile, forkhead box L2 (FOXL2) has been known as a winged-helix/forkhead (FH) domain transcription factor, and today, a variety of research on FOXL2 is progressing. That is, as a result of the research conducted by the Europe Molecular Biology Laboratory in 2010, in the thesis disclosed in the prominent scientific journal, *Cell,* a result of the research overturned the scientific fact that sex is determined only by XY chromosomes. Researchers confirmed the result in that the ovary of a female mouse with a knocked-out FOXL2 gene turned into a tissue with a testicle-like structure, and secreted testosterone, which is a male hormone, and in this process, particularly, confirmed that granulosa cells in the ovary gradually turned into sertoli cells which involve in maturation of sperm.

Further, as a result of the observation made on ovarian cells in which FOXL2 expression occurs and the step-by-step progression of the expression, it was confirmed that FOXL2 mRNA is expressed in ovaries of all of immature and mature mice from genesis, and particularly, expressed limitedly in undifferentiated granulosa cells present in small or medium-sized ovarian follicles, and thereby it was reported that FOXL2 is a factor regulating the growth of ovarian follicles.

Furthermore, according to the research result regarding the occurrence of the GCT of the ovary, disclosed in New England Journal of Medicine issued on June in 2009, it was reported that a point mutation (402C->G) occurred in a FOXL2 gene of 97% of the adult-type GCT patients, which was revealed through whole-transcriptome paired-end RNA sequencing. Accordingly, it was reported that single and recurrent somatic mutations (402C->G) of FOXL2 can be considered as latent regulators in the pathogenesis of adult-type GCTs (Refer to U. S. Patent Publication No. 2011/0195070).

The inventors confirmed that, by analysis of posttranslational modification of a FOXL2 protein, phosphorylation of FOXL2 serine 33 (S33) residue was detected, and, even when the C134W mutant was overexpressed, the degree of phosphorylation of the S33 residue was greater than that of the wild type (WT), they also found that GSK3beta was specifically involved in the phosphorylation of the FOXL2 S33 residue, and noted that a glycogen synthase kinase 3-beta (GSK3beta) inhibitory material can be effectively used to prevent, treat or improve the GCT of the ovary. Therefore, the present invention was completed based thereon. WO2012135588 mentions compound of Formula 1 (SB 216763) in studies for the treatment of ovarian cancer.
WO2006034207 discloses the compound of Formula 2 as GSK3 inhibitor in combinations for the treatment of hyperproliferative diseases, specifically ovarian cancer.

### [Disclosure]

### [Technical Problem]

The present invention is directed to providing a pharmaceutical composition for preventing or treating a GCT of the ovary, in which the composition contains one selected from the group consisting of a compound represented by Formula 1, a pharmaceutically acceptable salt of the compound represented by Formula 1, a compound represented by Formula 2, and a pharmaceutically acceptable salt of the compound represented by Formula 2, as an active ingredient.

In addition, the present invention is directed to providing a functional health food composition for preventing or improving a GCT of the ovary, in which the composition contains one selected from the group consisting of a compound represented by Formula 1, a pharmaceutically acceptable salt of the compound represented by Formula 1, a compound represented by Formula 2, and a pharmaceutically acceptable salt of the compound represented by Formula 2, as an active ingredient.

However, technical objects to be accomplished by the present invention are not limited to the objects disclosed above, and other objects not disclosed herein will be more clearly understood to those of ordinary skill in the art upon reading the following descriptions.

### [Technical Solution]

The present invention provides a pharmaceutical composition for preventing or treating a GCT of the ovary, the composition containing one selected from the group consisting of a compound represented by Formula 1, a pharmaceutically acceptable salt of the compound represented by Formula 1, a compound represented by Formula 2, and a pharmaceutically acceptable salt of the compound represented by Formula 2, as an active ingredient.

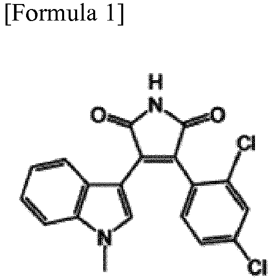

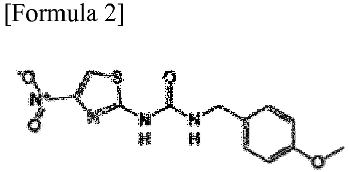

According to an exemplary embodiment of the present invention, the composition inhibits GSK3beta.

According to another exemplary embodiment of the present invention, the composition inhibits the phosphorylation of a serine which is the 33^{rd} amino acid of a FOXL2 protein.

In another aspect, the present invention provides a functional health food composition for preventing or improving a GCT of the ovary, the composition containing one selected from the group consisting of a compound represented by Formula 1, a pharmaceutically acceptable salt of the compound represented by Formula 1, a compound represented by Formula 2, and a pharmaceutically acceptable salt of the compound represented by Formula 2, as an active ingredient.

According to an exemplary embodiment of the present invention, the composition inhibits GSK3beta.

According to another exemplary embodiment of the present invention, the composition inhibits the phosphorylation of a serine which is the 33^{rd} amino acid of a FOXL2 protein.

### [Advantageous Effects]

A composition according to the present invention contains one selected from the group consisting of a compound represented by Formula 1, a pharmaceutically acceptable salt of the compound represented by Formula 1, a compound represented by Formula 2, and a pharmaceutically acceptable salt of the compound represented by Formula 2, as an active ingredient, and inhibits GSK3beta, thereby having an effect of inhibiting the phosphorylation of the 33^{rd} amino acid, serine, of a FOXL2 protein, and thus can be effectively used as a composition for preventing or treating a GCT of the ovary. In addition, the composition is expected to be effectively used as a functional health food composition.

### [Description of Drawings]

FIG. 1 is a diagram showing the result of sequence conservation analysis on FOXL2 S33 residues between non-mammals and mammals.
FIG. 2 is a diagram showing the result of confirming the degree of phosphorylation using western blotting performed to each of a cloned S33A mutant which is a non-phosphorylated mutant of FOXL2 and a cloned S33D mutant which is an overphosphorylated mutant.
FIG. 3 is a diagram showing the expected result for a kinase involved in phosphorylation using a GPS2.1 phosphoplot.
FIG. 4 is a diagram showing the western blotting result to confirm a kinase involved in the phosphorylation of the FOXL2 S33 residue.
FIG. 5 is a diagram showing the western blotting result to confirm whether GSK3beta specifically involves the phosphorylation of the FOXL2 S33 residue.
FIG. 6 is a diagram showing the western blotting result to confirm stable overexpression of FOXL2 and an S33-specific GSK3beta effect.
FIG. 7 is a diagram showing the result of an immunoprecipitation assay to detect binding domains of GSK3beta and a FOXL2 protein.
FIG. 8 is a diagram showing the western blotting result to detect the degree of S33 phosphorylation of FOXL2 C134W (GCT).
FIG. 9 is a diagram showing the result of an experiment to confirm the relationship of direct phosphorylation between FOXL2 C134W and an S33 residue.
FIG. 10 is a diagram showing the result of immunoprecipitation assay to confirm the GSK3beta-binding degree between a FOXL2 C134W mutant and the WT.
FIG. 11 is a diagram showing results of a luciferase assay and a cell viability assay to verify a GSK3beta inhibitory effect.
FIG. 12 is a diagram showing the result of an experiment for protein stability to verify the GSK3beta inhibitory effect.
FIG. 13 is a diagram showing the result of a test for a cell growth rate to verify the GSK3beta inhibitory effect.
FIG. 14 is a diagram showing the result of the verification of the efficacy of a GSK3beta inhibitory material to ovarian GCT cells.

### [Modes of the Invention]

Hereinafter, the present invention will be described in detail.

The present invention provides a pharmaceutical composition for preventing or treating a GCT, the composition containing one selected from the group consisting of 3-(2,4-Dichlorophenyl)-4-(1-methyl-1H-indol-3-yl)-1H-pyrrole-2,5-dione represented by Formula 1, a pharmaceutically acceptable salt of the compound represented by Formula 1, N-[(4-Methoxyphenyl)Methyl]-N'-(5-nitro-thiazol-2-yl)urea represented by Formula 2, and a pharmaceutically acceptable salt of the compound represented by Formula 2, as an active ingredient.

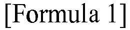

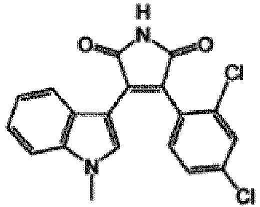

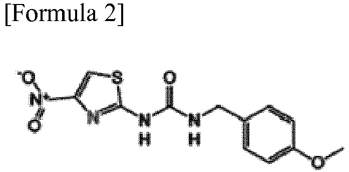

Each of the compounds represented by Formula 1 and Formula 2 may be prepared by a known chemical synthesis method, or used by purchased as a commercially-available reagent.

According to an exemplary embodiment of the present invention, it was confirmed that GSK3beta specifically regulates the phosphorylation of a FOXL2 S33 residue (refer to Example 4), when GSK3beta is inhibited using the compound represented by Formula 1 or 2, the phosphorylation of the FOXL2 S33 residue is decreased (refer to Example 5), the cell growth rate of a C134W mutant prominently found in GCT cells is decreased (refer to Example 9), and a size of the tumor induced in the GCT cells is decreased (refer to Example 10).

In addition, the compound represented by Formula 1 or 2 may be used in the form of a pharmaceutically acceptable salt, and the term "pharmaceutically acceptable salt" used herein refers to a form of a compound which does not induce serious stimulation to an organism into which the compound is administered, and does not degrade a biological activity and physical properties of the compound.

As the pharmaceutically acceptable salt, an acid addition salt formed by a pharmaceutically acceptable free acid is useful. The acid addition salt is obtained from an inorganic acid such as hydrochloric acid, nitric acid, phosphoric acid, sulfuric acid, hydrobromic acid, hydroiodic acid, azilic acid or phosphorous acid, or a non-toxic organic acid such as aliphatic mono or dicarboxylate, phenyl-substituted alkanoate, hydroxyalkanoate or alkanedioate, an aromatic acid, aliphatic or aromatic sulfonic acid. Such pharmaceutically non-toxic salt may be, but is not limited to, sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzene sulfonate, toluene sulfonate, chlorobenzene sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2-sulfonate, or mandelate.

The acid addition salt according to the present invention may be prepared by a conventional method, for example, by dissolving the compound represented by Formula 1 or 2 in an excessive amount of an acid aqueous solution, and precipitating the salt using a water-miscible organic solvent, for example, methanol, ethanol, acetone or acetonitrile. The acid addition salt may be prepared by heating equal amounts of the compound represented by Formula 1 or 2 and an acid or alcohol in water, and drying the mixture through evaporation or suction-filtering a precipitated salt.

In addition, a pharmaceutically acceptable metal salt may be prepared using a base. An alkali metal or alkali earth metal salt is obtained by, for example, dissolving a compound in an excessive amount of an alkali metal hydroxide or alkali earth metal hydroxide solution, filtering an undissolved compound salt, and evaporating and drying the filtrate. Here, the metal salt considered suitable for pharmaceutical use is a sodium salt, a potassium salt or a calcium salt. Further, a silver salt corresponding thereto is obtained by reacting a salt of an alkali metal or alkali earth metal with a suitable silver salt (e.g., silver nitrate).

The term "prevention" used herein refers to all types of behaviors involved in inhibition of a GCT of the ovary or delay of the occurrence of a GCT of the ovary by administration of the composition of the present invention.

The term "treatment" used herein refers to all behaviors involved in alleviation or beneficial change of the symptoms of a GCT of the ovary due to the administration of the composition of the present invention.

The pharmaceutical composition according to the present invention is prepared in a suitable form for administration by adding a generally used diluent or excipient such as a filler, an extender, a binder, a wetting agent, a disintegrating agent or a surfactant.

Solid preparations for oral administration are prepared in the form of a tablet, a pill, a powder, a granule, a capsule, or a troche, and such a solid preparation is prepared by mixing at least one excipient, for example, starch, calcium carbonate, sucrose or lactose or gelatin to at least one compound according to the present invention. In addition, lubricants such as magnesium stearate talc may be added, in addition to the simple excipient. A liquid preparation for oral administration may be prepared in the form of a suspending agent, an oral liquid, an emulsion or a syrup, and may contain various excipients, for example, a wetting agent, a sweetening agent, an aromatic agent and a preservative, in addition to a frequently used simple diluent such as water or liquid paraffin.

A preparation for parenteral administration is prepared in the form of a sterilized aqueous solution, a non-aqueous solvent, a suspending agent, an emulsion, a lyophilized preparation, or a suppository.

As a non-aqueous solvent or a suspending agent, propylene glycol, polyethylene glycol, a vegetable oil such as an olive oil, or an injectable ester such as ethyloleate may be used. As the base material for the suppository, witepsol, macrogol, tween 61, cacao butter, laurinum, glycerol or gelatin may be used.

The composition of the present invention may be administered orally or parenterally (for example, intravenously, subcutaneously, intraperitoneally or locally administered) depending on a desired method, and although a dose of the composition varies depending on a condition and body weight of a patient, the severity of a disease, a dosage form, and an administration route and time, it can be appropriately determined by those of ordinary skill in the art.

The composition according to the present invention is administered in a pharmaceutically effective amount. The term "pharmaceutically effective amount" used herein refers to an amount sufficient to treat the disease at a reasonable benefit/risk ratio applicable for medical treatment, and the level of an effective dosage may be determined by parameters including a type of illness of a patient, severity, the activity of a drug, sensitivity to a drug, administration time, an administration route and a release rate, duration of treatment, co-used drugs, and other parameters well known in medical fields. The composition of the present invention may be administered alone as an individual therapeutic agent or in combination with a different therapeutic agent, administered sequentially or simultaneously with a conventional therapeutic agent, or administered in a single or multiple dose regime. In consideration of all of the above factors, it is important to administer such a dose as to obtain a maximum effect with a minimal amount without a side effect and the dose may be easily determined by those of ordinary skill in the art.

Specifically, the effective amount of the compound according to the present invention may vary depending on the age, sex or body weight of a patient, and the compound may be generally administered at 0.001 to 150 mg, and preferably, 0.01 to 100 mg per 1 kg of body weight, daily or every other day, or once to three times a day. However, the effective amount may vary depending on an administration route, the severity of obesity, sex, body weight or age of a patient, and therefore, it should be noted that the present invention is not limited by the dose.

The present invention provides a functional health food composition for preventing or improving a GCT of the ovary, the composition containing one selected from the group consisting of a compound represented by Formula 1, a pharmaceutically acceptable salt of the compound represented by Formula 1, a compound represented by Formula 2, and a pharmaceutically acceptable salt of the compound represented by Formula 2, as an active ingredient. That is, to prevent or improve a GCT of the ovary, the composition of the present invention may be used alone or in combination of a drug for treating a tumor before or after the occurrence of the GCT of the ovary.

The term "improvement" used herein refers to all of the behaviors involved in reduction of a parameter related to a treated state, for example, the severity of a symptom.

Since the composition for a functional health food according to the present invention inhibits GSK3beta, and thus inhibits the phosphorylation of a serine which is the 33^{rd} amino acid of a FOXL2 protein, the composition may be added to a health food supplement such as a food or drink to prevent or improve a GCT of the ovary.

A type of the food is not particularly limited. Examples of the food to which the composition can be added may include drinks, meats, sausages, bread, biscuits, rice cakes, chocolates, candies, snacks, cookies, pizza, ramen, other noodles, gum, dairy products including ice creams, various types of soup, beverages, alcoholic beverages and vitamin complexes, milk products and processed dairy products, and include all functional health foods in the common sense.

The composition of the present invention may be added to a food alone or in combination with a different food or a food ingredient, and may be suitably used according to a conventional method. The amount of the active ingredient to be mixed may be appropriately determined depending on a purpose (prevention or improvement) of using the active ingredient. Generally, in manufacturing of a food or beverage, the composition of the present invention is added at 15 wt% or less, and preferably, 10 wt% or less with respect to a base material. However, in the case of long-term intake for health and hygiene or health control, the amount may be less than the above range.

The composition for a health beverage of the present invention may contain the above-described compound as an essential ingredient at a predetermined ratio, and various flavoring agents or natural carbohydrates as additional ingredients without particular limitation, like a conventional beverage. Examples of the above-described natural carbohydrates include conventional sugars such as a monosaccharide, for example, glucose, fructose, etc.; a disaccharide, for example, maltose, sucrose, etc.; and a polysaccharide, for example, dextrin, cyclodextrin, etc. and sugar alcohols such as xylitol, sorbitol, erythritol, etc. In addition to the above-described ingredients, a natural flavoring agent (thaumatin, a stevia extract (e.g., rebaudioside A, glycyrrhizin, etc.) and a synthetic flavoring agent (saccharin, aspartame, etc.) may be favorably used as the flavoring agent. A ratio of the natural carbohydrate may be suitably determined by choice of those of ordinary skill in the art.

In addition to the above-described ingredients, the composition for a functional health food of the present invention may further contain various nutritional supplements, vitamins, minerals (electrolytes), flavoring agents such as a synthetic flavoring agent and a natural flavoring agent, a coloring agent and an enhancer (cheese, chocolate, etc.), pectic acid and a salt thereof, alginic acid and a salt thereof, an organic acid, a protective colloid thickener, a pH regulator, a stabilizer, a preservative, glycerin, an alcohol, a carbonating agent used in soft drinks. In addition, the composition of the present invention may contain a natural fruit juice, and flesh for manufacturing fruit juice drinks and vegetable drinks. Such an ingredient may be used independently or in combination with other ingredients. A ratio of the additive may also be suitably selected by those of ordinary skill in the art.

Hereinafter, exemplary examples will be provided to help the understanding of the present invention. However, the examples disclosed below are merely provided to facilitate the understanding of the present invention, not to limit the scope of the present invention.

### [Examples]

### Example 1. Preparation for experiment

### 1-1. Cloning of FOXL2 mutant

To clone various FOXL2 mutant types, recombinant PCR was performed using the primers listed in Table 1, and an amplified PCR product was transformed in E. coli through ligation to a pCMV-Myc vector using EcoRI and XhoI restriction enzymes. Meanwhile, FOXL2 C134W+S33A mutant was cloned using a C134W mutant as a template and S33A forward and reverse primers.

**[Table 1]**

| Mutant-type | Forward primer(5'→3') | | Reverse primer(5'→3') | |
|---|---|---|---|---|
| FOXL2 | SEQ. ID. NO: 1 | | SEQ. ID. NO: 2 | |
| S33A | SEQ. ID. NO: 3 | | SEQ. ID. NO: 4 | |
| S33D | SEQ. ID. NO: 5 | | SEQ. ID. NO: 6 | |
| S263A | SEQ. ID. NO: 7 | | SEQ. ID. NO: 8 | |
| K25R | SEQ. ID. NO: 9 | | SEQ. ID. NO: 10 | |
| K36R | SEQ. ID. NO: 11 | | SEQ. ID. NO: 12 | |
| K48R | SEQ. ID. NO: 13 | | SEQ. ID. NO: 14 | CGGGTCCGGTCTCTCCGGGGC |
| K54R | SEQ. ID. NO: 15 | | SEQ. ID. NO: 16 | GGGTCTCTGCGCCGGGTCCGG |
| K87R | SEQ. ID. NO: 17 | | SEQ. ID. NO: 18 | GAACGGGAATCTCGCGATGAT |
| C134W | SEQ. ID. NO: 19 | | SEQ. ID. NO: 20 | ATGTCTTCCCAGGCCGGGTC |

### 1-2. Cell culture

For an experiment of the present example, human granulosa (KGN) cells were cultured in DMEM/F12 media containing 10% FBS and 1% penicillin-streptomycin. The KGN cells were provided by Yosihiro Nishi and Toshihiko Yanase.

### 1-3. Construction of cell lines

To construct clones for stably expressing FOXL2 in cells, FOXL2 WT (wild type), S33D, S33A and C134W constructs were ligased to a pcDNA6 plasmid using EcoRI and XhoI restriction enzymes for cloning. Afterward, 10 µg of DNA was transfected to 1×10⁷ of the KGN cells, and then selected using blasticidin for 3 to 4 weeks.

### 1-4. Transfection for overexpression of protein in cells

For overexpression of a protein by introducing a plasmid into cells, a plasmid to be introduced was introduced at 3 µg per 1×10⁶ KGN cells, and for the introduction, transfection was performed using a Neon® electro transfection kit, which is an In vitro transfection kit.

### Example 2. Methods for experiment

### 2-1. Western blotting

The cultured cells were harvested, and a protein was extracted from the cells using a Nonidet P-40 (NP-40) solution. For quantification of the extracted protein, a Bicinchoninic acid (BCA)™ protein assay was performed. The quantified protein was transferred to a polyvinylidene fluoride (PVDF) membrane by SDS-PAGE electrophoresis, and incubated with an antibody for each protein to be detected. Afterward, the proteins were detected using secondary antibodies and a ChemDoc system.

### 2-2. Immunoprecipitation

Cells were sampled and lysed in an NP-40 lysis buffer, and a protein amount was measured. Afterward, an antibody of a specific protein to be precipitated and a normal IgG of an antibody as a control were independently cultured, and then cultured with proteinase agarose G binding to an antibody. The cultured sample and beads were washed with NP-40 buffer three times and loaded using western blotting, and thereby a band was detected using an antibody of a different protein to be tested.

### 2-3. Ubiquitination and SUMOylation

The overexpressed sample was treated with 50 µM of a proteosome inhibitor, that is, MG132, and cultured, and then a protein was sampled with NP-40 buffer. Afterward, an overexpressed protein was precipitated through immunoprecipitation, and the sample was loaded and reacted with an ubiquitin antibody and a SUMO antibody using western blotting, thereby detecting a band using a Chemdoc system.

### 2-4. Statistical analysis

Each experiment was run in triplicate, and then mean and standard deviations were calculated. In each experiment, statistical analysis was performed according to a Student's t-test using an SAS statistical software (SAS Enterprise Guide, USA), and a p value of p<0.05 was interpreted to be significant.

### Example 3. Analysis of phosphorylation of FOXL2 protein

According to the result of the analysis of posttranslational modification of FOXL2 protein using liquid chromatography, the phosphorylation of a serine which is the 33^{rd} amino acid of FOXL2 was confirmed.

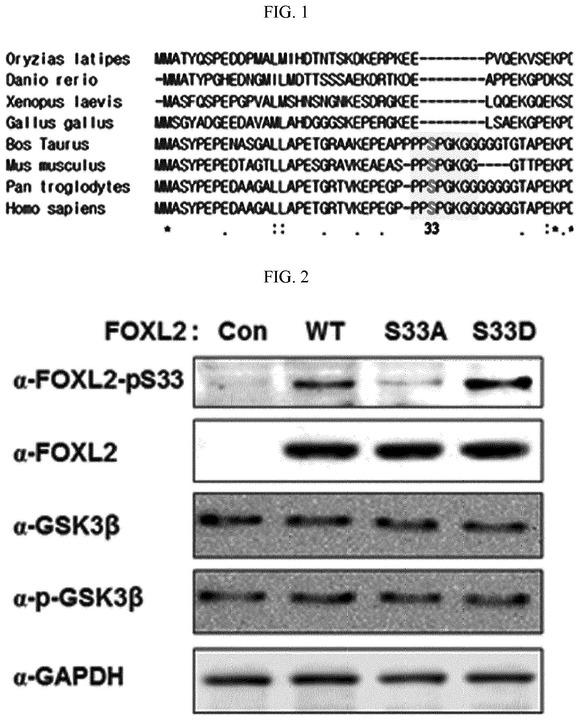

In addition, in order to confirm the significance of a FOXL2 S33 residue in evolution, FOXL2 was sequenced for a comparative sequence analysis between species, and the result is shown in FIG. 1.

As shown in FIG. 1, it was confirmed that the S33 residue is located conservatively in mammals.

Meanwhile, to perform an experiment on the phosphorylation of the FOXL2 S33 residue, S33A which is the non-phosphorylated mutant of FOXL2 and S33D which is the overphosphorylated mutant of FOXL2 were cloned, and the degree of phosphorylation for each mutant was measured according to western blotting, and the result is shown in FIG. 2.

As shown in FIG. 2, in the non-phosphorylated mutant, the band of the FOXL2 S33 residue was not detected, and in the overphosphorylated mutant, a stronger band than that in the WT was detected, and therefore, it was confirmed that the FOXL2 S33 residue was overphosphorylated.

### Example 4. Prediction and confirmation of kinase involved in phosphorylation of FOXL2 S33 residue

### 4-1. Prediction of kinase involved in phosphorylation of FOXL2 S33 residue

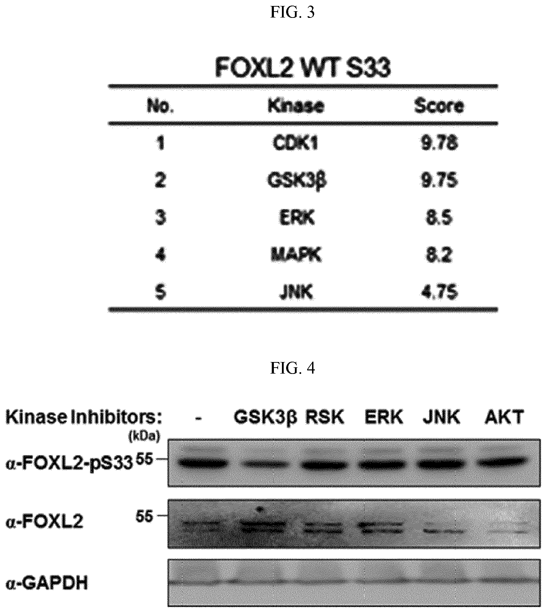

To assay a kinase involved in the phosphorylation of the FOXL2 S33 residue confirmed to be conservative in mammals according to Example 3, a kinase involved in phosphorylation was expected using a prediction program, that is, a GPS2.1 phosphoplot, and the result is shown in FIG. 3.

As shown in FIG. 3, it was confirmed that a score of GSK3beta was 9.75. Accordingly, it was expected from the result that the kinase can be involved in the phosphorylation of the FOXL2 S33 residue.

### 4-2. Confirmation of kinase involved in phosphorylation of FOXL2 S33 residue

First, the kinase expected according to Example 4-1 and another kinase known to be involved in phosphorylation were prepared. Specifically, a GSK3beta inhibitor (SB 216763), an RSK inhibitor (SL0101), an ERK inhibitor (U0126), a JNK inhibitor (SP600125) and an AKT inhibitor (LY 294002) were prepared, and among those, the GSK3beta inhibitor, that is, SB 216763, contains a compound represented by Formula 1.

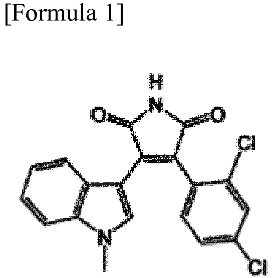

While the FOXL2 WT was overexpressed in the KGN cells, the cells were treated with 10 µM of the inhibitor of each kinase, cultured for 18 hours and sampled, and then a change in phosphorylation was measured by performing western blotting. Here, the change in phosphorylation was measured using an antibody specifically binding to a peptide consisting of an amino acid of SEQ. ID. NO: 21, and the result is shown in FIG. 4.

As shown in FIG. 4, it was confirmed that, when treated with the GSK3beta inhibitor, the phosphorylation of the FOXL2 S33 residue was reduced. Accordingly, it can be noted from the result that GSK3beta was involved in the phosphorylation of the FOXL2 S33 residue.

### Example 5. Confirmation of role of GSK3beta specifically regulating phosphorylation of FOXL2 S33 residue

To confirm whether an inhibitory effect of GSK3beta on the FOXL2 S33 residue is a GSK3beta-specific effect, cells in which FOXL2 WT was overexpressed were treated with 10 µM each of AR-A014418 and TWS1119, which are two different GSK3beta inhibitors, sampled, and analyzed by western blotting. Here, AR-A014418 contains a compound represented by Formula 2.

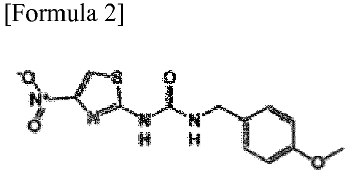

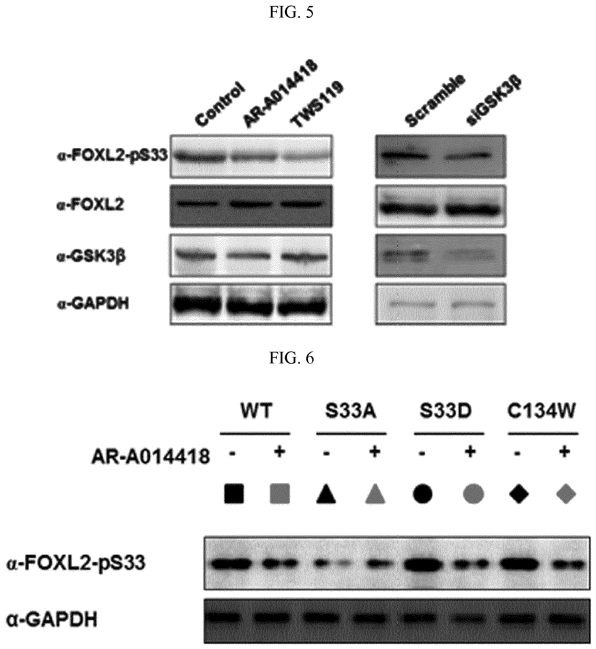

Moreover, when GSK3beta was knocked-down by treatment with GSK3beta-specific siRNA, changes in the phosphorylation of the FOXL2 S33 residue was detected through western blotting, and the result is shown in FIG. 5.

As shown in FIG. 5, it was confirmed that, when the GSK3beta inhibitor was used, the phosphorylation of the FOXL2 S33 residue was reduced.

In addition, in order to confirm a regulatory effect of GSK3beta on the phosphorylation of the FOXL2 S33 residue, KGN cells, one type of human ovarian granulosa cells, in which FOXL2 was stably overexpressed, were constructed. Cells in which FOXL2 WT, S33D, S33A and C134W were stably expressed were treated with 10 nM of GSK3beta, and the degree of phosphorylation of FOXL2 was measured through western blotting, and the result is shown in FIG. 6.

As shown in FIG. 6, it was confirmed that GSK3beta regulates the phosphorylation of the FOXL2 S33 residue also in cell lines in which FOXL2 is stably expressed.

Accordingly, it can be noted from the result that GSK3beta specifically phosphorylates the FOXL2 S33 residue.

### Example 6. Confirmation of binding domains of GSK3beta and FOXL2 proteins

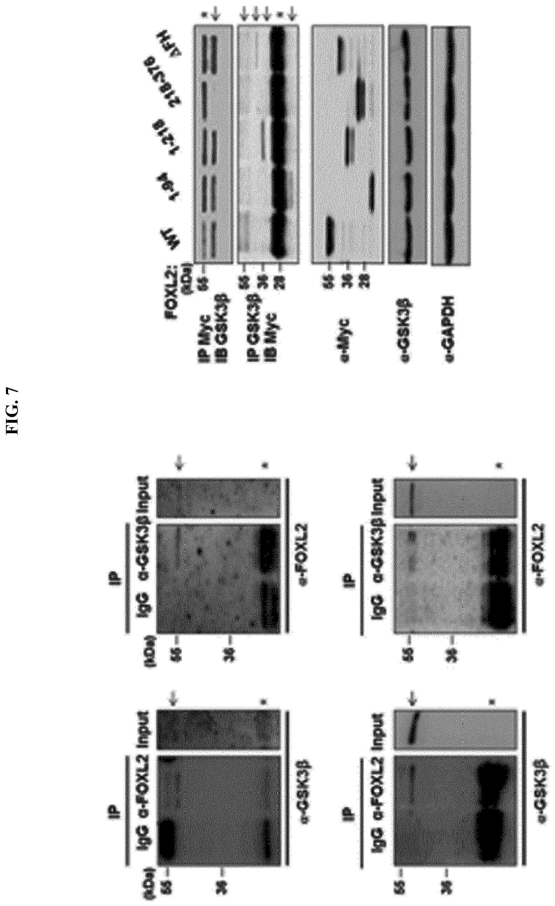

To confirm whether GSK3beta directly binds to FOXL2 for phosphorylation, KGN cells which are ovarian granulosa cells were sampled, and the binding between the FOXL2 and GSK3beta protein was detected using immunoprecipitation. In addition, to detect the binding domains of the two proteins, the binding to deletion mutant proteins of FOXL2 (1-94 a.a, 1-218 a.a, 218-376 a.a. deletion forkhead domain) was detected using immunoprecipitation, and the result is shown in FIG. 7.

As shown in FIG. 7, it can be confirmed that FOXL2 and GSK3beta proteins directly bound to each other, and the binding occurred at an N-terminal domain.

### Example 7. Confirmation of degree of phosphorylation in GCT cells

To examine the relationship between the phosphorylation of the FOXL2 S33 residue and a disease caused by FOXL2, the following experiment was performed.

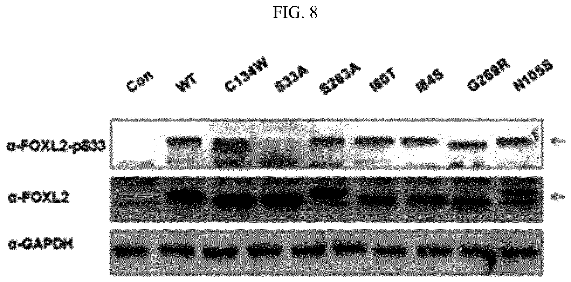

That is, the overexpression of the FOXL2 mutants constructed in Example 1 was induced, the degree of the phosphorylation of the FOXL2 S33 residue was detected through western blotting, and the result is shown in FIG. 8.

As shown in FIG. 8, it can be confirmed that when the C134W mutant, which was recently reported to be found in 97% of the patient with GCT of the ovary, was overexpressed, the S33 residue was more highly phosphorylated than that in the WT(wild type).

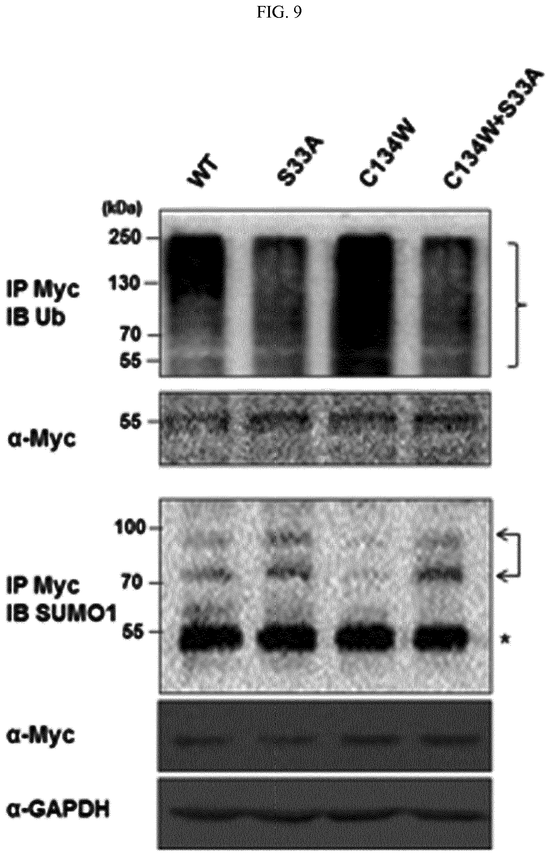

In addition, to examine the relationship between the FOXL2 C134W mutant and the phosphorylation of the S33 residue, patterns of ubiquitination and sumoylation were examined according to the method described in Example 2-3, and the result is shown in FIG. 9.

As shown in FIG. 9, it can be confirmed that ubiquitination was increased and sumoylation was decreased in the FOXL2 C134W mutant. However, such a change was not detected in a C134W sample having S33A mutation.

Based on the result obtained above, it was found that overphosphorylation of the FOXL2 C134W mutant is directly related to the S33 residue.

### Example 8. Confirmation of GSK3beta binding degree between FOXL2 C134W mutant and WT

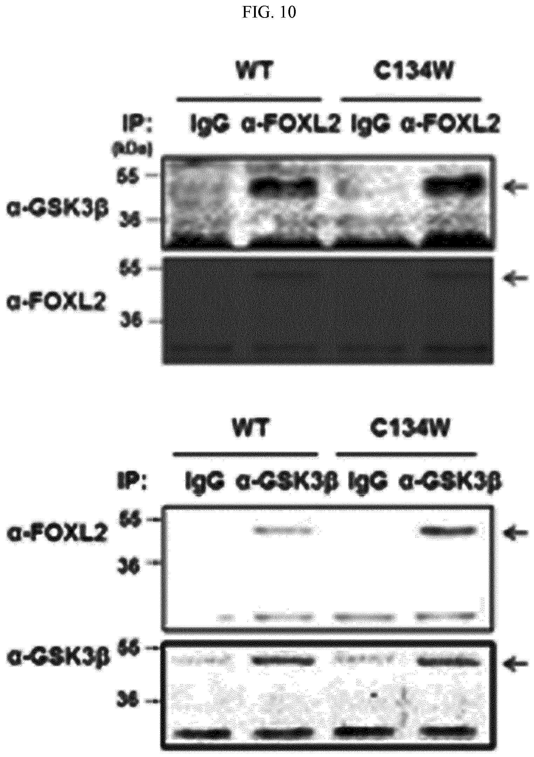

To confirm the relationship between the FOXL2 C134W protein which is GCT-induced mutant and GSK3beta, the degree of GSK3beta binding between the FOXL2 C134W mutant and the WT was confirmed using immunoprecipitation, and the result is shown in FIG. 10.

As shown in FIG. 10, it can be confirmed that the C134W mutant more highly bound to GSK3beta than the WT.

### Example 9. Verification of effect of inhibiting GSK3beta

### 9-1. Luciferase assay and cell viability assay

To verify an effect brought by inhibition of GSK3beta regulating the phosphorylation of FOXL2, a luciferase assay for proteins of FOXL2 target genes such as TNFR1, FAS, Caspase 8 and a cell viability assay were performed.

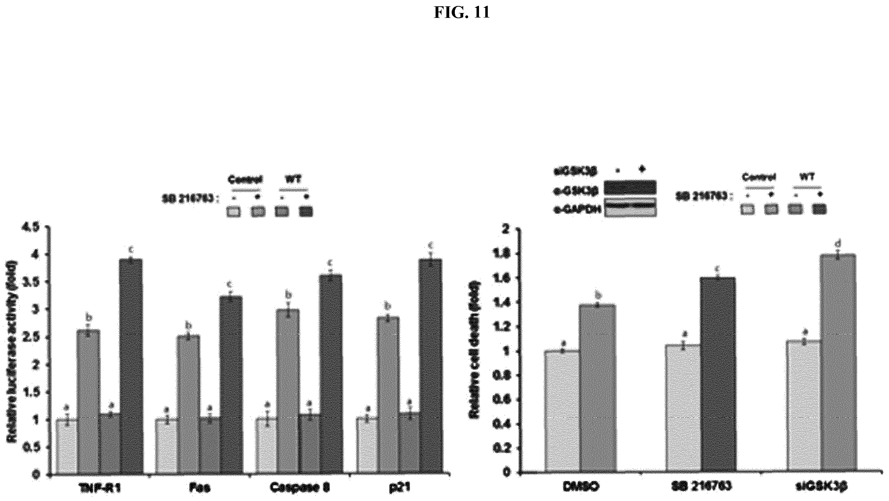

Specifically, the luciferase assay was performed by transfecting 4×10⁵ per well of the KGN cells with 170 ng of pCMV-galactosidase plasmid DNA, 300 ng of reporter DNA of TNFR1, FAS, Caspase 8 amd p21, FOXL2 or a mutant protein plasmid in a 6-well plate using a Microporator MP-100, culturing for 20 hours, and measuring absorbance using a luciferase kit provided by Promega and Perkin Elmer 1420 counter, and the result is shown in FIG. 11.

In addition, for the cell viability assay, the KGN cells were cultured at a density of 2×10⁴ per well in a 96 well plate after overexpression of a plasmid expressing a specific protein. Afterward, cell viability was measured using a CellTiterGlo cell viability kit provided by Promega and a Perkin Elmer 1420 counter, and the result is shown in FIG. 11.

As shown in FIG. 11, it can be confirmed that when GSK3beta was inhibited or absent, the phosphorylation of FOXL2 was inhibited, thereby activating TNFR1, FAS, Caspase8 and p21 (when the expression levels of these genes were high, cell viability was decreased, leading to an increase in cell death) promoters, and thus the cell viability was decreased.

### 9-2. Experiment for protein stability

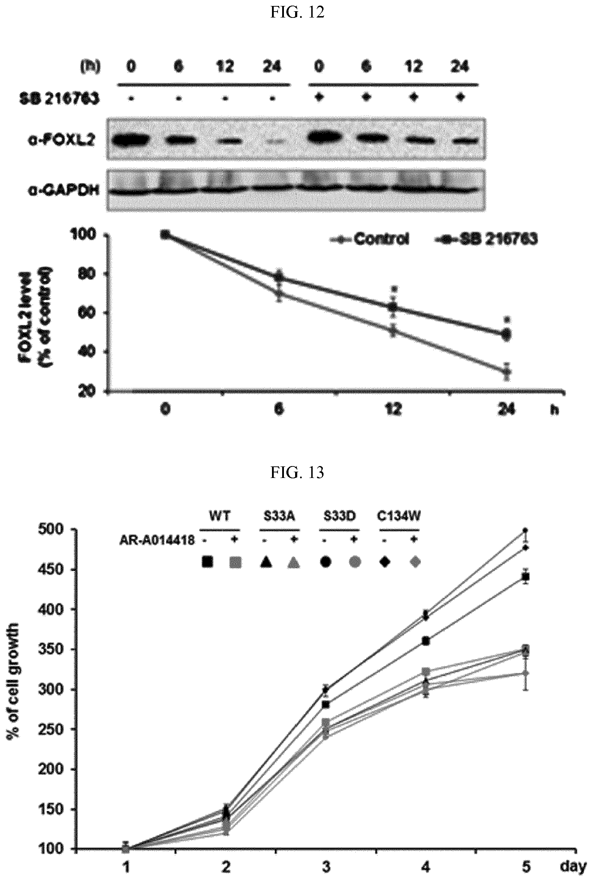

To confirm whether the effect of decreasing the cell viability by inducing the activity of a specific promoter such as TNFR1, FAS, Caspase8 and p21 induces regulation of FOXL2 protein stability by the phosphorylation of the FOXL2 S33 residue, cells in which a FOXL2 protein was overexpressed were treated with a GSK3beta inhibitor (SB 216763) and expressed, treated with 10 µg of cyclohexamide which is a protein synthesis inhibitor, and then sampled at 0, 6, 12, and 24 hours to perform western blotting, and the result is shown in FIG. 12.

As shown in FIG. 12, it can be confirmed that, when GSK3beta was inhibited, the protein stability was increased.

### 9-3. Experiment for cell growth rate

After the cells in which the FOXL2 protein was overexpressed were treated with a GSK3beta inhibitor (AR-A014418), a cell growth rate according to time was measured, and the result is shown in FIG. 13.

As shown in FIG. 13, it can be confirmed that, when GSK3beta was inhibited or absent, the phosphorylation of FOXL2 was inhibited, leading to a decrease in cell growth rate, and particularly, the cell growth rate of the C134W mutant which was predominantly found in a GCT, was remarkably decreased.

### Example 10. Verification of efficacy of GSK3beta inhibitory material to ovarian GCT cells

To verify an efficacy of a GSK3beta inhibitory material in GCT, the following experiment was performed.

6-week-old mature male BALB/c-nu mice were purchased from Central Laboratory Animal, Inc., and allowed for one week to be adapted to environments of the breeding facility and the laboratory in the Chung-Ang University Laboratory Animal Research Center to ensure that the mice had no general symptoms, prior to being used for the experiment. The animals were housed at a density of five mice per polycarbonate cage, and solid food for laboratory animals and water were freely provided. The environments for the breeding laboratory including a temperature, humidity, a ventilation cycle, a light/dark cycle, and illuminance were constantly maintained, and all the experiments were conducted in accordance with the regulation of the Chung-Ang University Animal Research Ethics Committee.

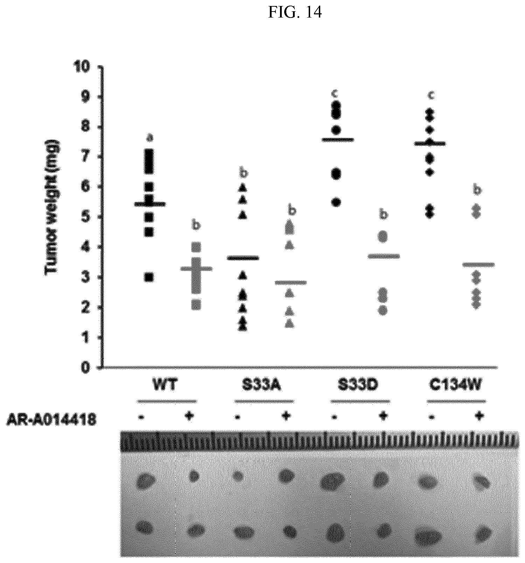

3×10⁷ of KGN cells stably expressing FOXL2 were subcutaneously injected into each mouse to induce a tumor. Three weeks after the injection, the GSK3beta inhibitor (AR-A014418) was directly injected into a tumor-induced region at 2 mg/kg 10 times for two weeks and then a tumor size was measured, and the result is shown in FIG. 14.

As shown in FIG. 14, it can be confirmed that a size of the tumor induced in the ovarian GCT cells was remarkably decreased in a GSK3beta inhibitor-treated group.

In addition, it was confirmed from the nude mouse models that the GSK3beta inhibitory material can substantially inhibit tumor production in ovarian granulosa cells in vivo.

From the result obtained above, it can be noted from the result that the GSK3beta inhibitory material had an excellent effect for preventing or treating GCT.

The above descriptions of the present invention are to explain the present invention, and it will be understood by those of ordinary skill in the art that the exemplary embodiments can be easily modified into different forms without changing the technical idea or essential characteristics of the present invention. Therefore, it should be interpreted that the exemplary embodiments described above are exemplary, but the present invention is not limited to the embodiments.

### [Industrial Applicability]

A composition according to the present invention inhibits the phosphorylation of a serine which is the 33^{rd} amino acid of a FOXL2 protein by inhibiting GSK3beta, and thus can be useful as a composition for preventing or treating GCT.
## Claims

1. A pharmaceutical composition for use in preventing or treating a granulosa cell tumor of the ovary, comprising:
one selected from the group consisting of a compound represented by Formula 2, a pharmaceutically acceptable salt of the compound represented by Formula 2, a compound represented by Formula 1, and a pharmaceutically acceptable salt of the compound represented by Formula 1, as an active ingredient.

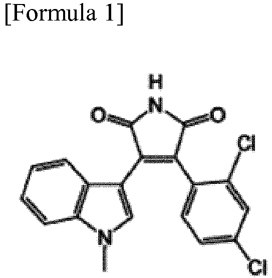

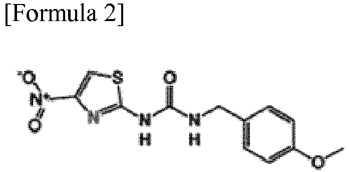

2. The pharmaceutical composition for use as claimed in claim 1, wherein the composition inhibits glycogen synthase kinase 3-beta (GSK3beta).

3. The pharmaceutical composition for use as claimed in claim 1 or 2, wherein the composition inhibits phosphorylation of a serine which is the 33^{rd} amino acid of a forkhead box L2 (FOXL2) protein.

4. A functional health food composition for use in preventing or improving a granulosa cell tumor of the ovary, comprising:
one selected from the group consisting of a compound represented by Formula 2, a pharmaceutically acceptable salt of the compound represented by Formula 2, a compound represented by Formula 1, and a pharmaceutically acceptable salt of the compound represented by Formula 1, as an active ingredient.

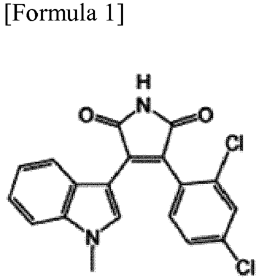

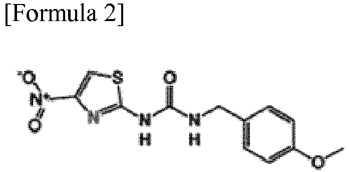

5. The food composition for use as claimed in claim 4, wherein the composition inhibits glycogen synthase kinase 3-beta (GSK3beta).

6. The food composition for use as claimed in claim 4 or 5, wherein the composition inhibits phosphorylation of a serine which is the 33^{rd} amino acid of a forkhead box L2 (FOXL2) protein.

## Patentansprüche

1. Pharmazeutische Zusammensetzung zur Verwendung bei der Prävention oder Behandlung eines Granulosazelltumors des Eierstocks, umfassend:
eines ausgewählt aus der Gruppe, bestehend aus einer durch Formel 2 dargestellten Verbindung, einem pharmazeutisch akzeptablen Salz der durch Formel 2 dargestellten Verbindung, einer durch Formel 1 dargestellten Verbindung und einem pharmazeutisch akzeptablen Salz der durch Formel 1 dargestellten Verbindung, als einen Wirkstoff.

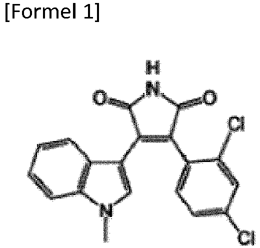

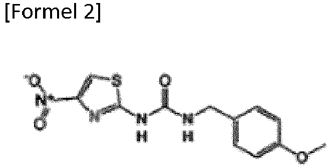

2. Pharmazeutische Zusammensetzung zur Verwendung nach Anspruch 1, wobei die Zusammensetzung Glycogen-Synthase-Kinase-3-beta (GSK3beta) hemmt.

3. Pharmazeutische Zusammensetzung zur Verwendung nach Anspruch 1 oder 2, wobei die Zusammensetzung Phosphorylierung eines Serins hemmt, das die 33. Aminosäure eines Forkhead-Box-L2-Proteins (FOXL2) ist.

4. Funktionelle Gesundheitsnahrungsmittelzusammensetzung zur Verwendung bei der Prävention oder Verbesserung eines Granulosazelltumors des Eierstocks, umfassend:
eines ausgewählt aus der Gruppe, bestehend aus einer durch Formel 2 dargestellten Verbindung, einem pharmazeutisch akzeptablen Salz der durch Formel 2 dargestellten Verbindung, einer durch Formel 1 dargestellten Verbindung und einem pharmazeutisch akzeptablen Salz der durch Formel 1 dargestellten Verbindung, als einen Wirkstoff.

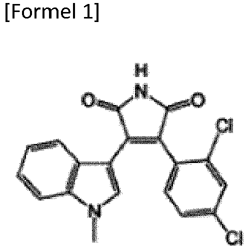

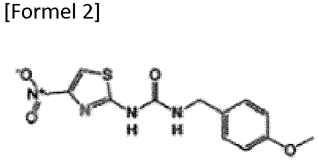

5. Nahrungsmittelzusammensetzung zur Verwendung nach Anspruch 4, wobei die Zusammensetzung Glycogen-Synthase-Kinase-3-beta (GSK3beta) hemmt.

6. Nahrungsmittelzusammensetzung zur Verwendung nach Anspruch 4 oder 5, wobei die Zusammensetzung Phosphorylierung eines Serins hemmt, das die 33. Aminosäure eines Forkhead-Box-L2-Proteins (FOXL2) ist.

## Revendications

1. Composition pharmaceutique à utiliser dans la prévention ou le traitement d'une tumeur de la granulosa de l'ovaire, comprenant :
un choisi dans le groupe constitué par un composé représenté par la formule 2, un sel pharmaceutiquement acceptable du composé représenté par la formule 2, un composé représenté par la formule 1 et un sel pharmaceutiquement acceptable du composé représenté par la formule 1, en tant que principe actif.

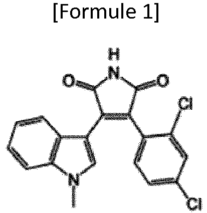

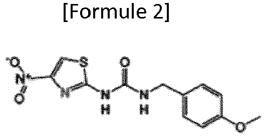

2. Composition pharmaceutique à utiliser selon la revendication 1, dans laquelle la composition inhibe la glycogène synthase kinase 3-bêta (GSK3beta).

3. Composition pharmaceutique à utiliser selon la revendication 1 ou 2, dans laquelle la composition inhibe la phosphorylation d'une sérine qui est le 33ème acide aminé d'une protéine FOXL2 (forkhead box L2).

4. Composition alimentaire diététique fonctionnelle destinée à être utilisée dans la prévention ou l'amélioration d'une tumeur de la granulosa de l'ovaire, comprenant :
un choisi dans le groupe constitué par un composé représenté par la formule 2, un sel pharmaceutiquement acceptable du composé représenté par la formule 2, un composé représenté par la formule 1 et un sel pharmaceutiquement acceptable du composé représenté par la formule 1, en tant que principe actif.

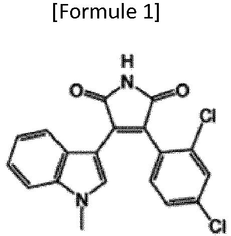

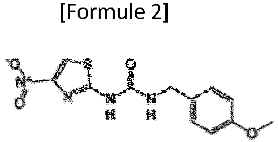

5. Composition alimentaire à utiliser selon la revendication 4, dans laquelle la composition inhibe la glycogène synthase kinase 3-bêta (GSK3beta).

6. Composition alimentaire à utiliser selon la revendication 4 ou 5, dans laquelle la composition inhibe la phosphorylation d'une sérine qui est le 33ème acide aminé d'une protéine FOXL2 (forkhead box L2).


<110> Chung-Ang University Industry-Academy Cooperation Foundation
<120> PHARMACEUTICAL COMPOSITION FOR PREVENTING OR TREATING OVARY GRANULOSA CELL TUMORS CONTAINING GLYCOGEN SYNTHASE KINASE-3 BETA INHIBITOR AS ACTIVE INGREDIENT, AND FUNCTIONAL HEALTH FOOD COMPOSITION
<130> PCTB01967
<150> 10-2013-0034672
   <151> 2013-03-29
<160> 21
<170> KopatentIn 2.0
<210> 1
   <211> 29
   <212> DNA
   <213> Artificial Sequence
<220>
   <223> FOXL2 primer F1
<400> 1
   ctagaattca aatgatggcc agctacccc 29
<210> 2
   <211> 30
   <212> DNA
   <213> Artificial Sequence
<220>
   <223> FOXL2 primer R1
<400> 2
   ctactcgagt cagagatcga ggcgcgaatg 30
<210> 3
   <211> 27
   <212> DNA
   <213> Artificial Sequence
<220>
   <223> FOXL2 S33A primer F1
<400> 3
   ccggccccag gcaagggcgg tgggggt 27
<210> 4
   <211> 27
   <212> DNA
   <213> Artificial Sequence
<220>
   <223> FOXL2 S33A primer R1
<400> 4
   acccccaccg cccttgcctg gggccgg 27
<210> 5
   <211> 24
   <212> DNA
   <213> Artificial Sequence
<220>
   <223> FOXL2 S33D primer F1
<400> 5
   ccgccggatc caggcaaggg cggt 24
<210> 6
   <211> 24
   <212> DNA
   <213> Artificial Sequence
<220>
   <223> FOXL2 S33D primer R1
<400> 6
   accgcccttg cctggatccg gcgg 24
<210> 7
   <211> 24
   <212> DNA
   <213> Artificial Sequence
<220>
   <223> FOXL2 S263A primer F1
<400> 7
   caggccatgg cgctgccccc cggc 24
<210> 8
   <211> 24
   <212> DNA
   <213> Artificial Sequence
<220>
   <223> FOXL2 S263A primer R1
<400> 8
   gccggggggc agcgccatgg cctg 24
<210> 9
   <211> 24
   <212> DNA
   <213> Artificial Sequence
<220>
   <223> FOXL2 K25R primer F1
<400> 9
   ggtcgcacag tcagagagcc agaa 24
<210> 10
   <211> 24
   <212> DNA
   <213> Artificial Sequence
<220>
   <223> FOXL2 K25R primer R1
<400> 10
   ttctggctct ctgactgtgc gacc 24
<210> 11
   <211> 24
   <212> DNA
   <213> Artificial Sequence
<220>
   <223> FOXL2 K36R primer F1
<400> 11
   ccaggcagag gcggtggggg tggc 24
<210> 12
   <211> 24
   <212> DNA
   <213> Artificial Sequence
<220>
   <223> FOXL2 K36R primer R1
<400> 12
   gccaccccca ccgcctctgc ctgg 24
<210> 13
   <211> 21
   <212> DNA
   <213> Artificial Sequence
<220>
   <223> FOXL2 K48R primer F1
<400> 13
   gccccggaga gaccggaccc g 21
<210> 14
   <211> 21
   <212> DNA
   <213> Artificial Sequence
<220>
   <223> FOXL2 K48R primer R1
<400> 14
   cgggtccggt ctctccgggg c 21
<210> 15
   <211> 21
   <212> DNA
   <213> Artificial Sequence
<220>
   <223> FOXL2 K54R primer F1
<400> 15
   ccggacccgg cgcagagacc c 21
<210> 16
   <211> 21
   <212> DNA
   <213> Artificial Sequence
<220>
   <223> FOXL2 K54R primer R1
<400> 16
   gggtctctgc gccgggtccg g 21
<210> 17
   <211> 21
   <212> DNA
   <213> Artificial Sequence
<220>
   <223> FOXL2 K87R primer F1
<400> 17
   atcatcgcga gattcccgtt c 21
<210> 18
   <211> 21
   <212> DNA
   <213> Artificial Sequence
<220>
   <223> FOXL2 K87R primer R1
<400> 18
   gaacgggaat ctcgcgatga t 21
<210> 19
   <211> 20
   <212> DNA
   <213> Artificial Sequence
<220>
   <223> FOXL2 C134W primer F1
<400> 19
   gcctgggaag acatgttcga 20
<210> 20
   <211> 20
   <212> DNA
   <213> Artificial Sequence
<220>
   <223> FOXL2 C134W primer R1
<400> 20
   atgtcttccc aggccgggtc 20
<210> 21
   <211> 11
   <212> PRT
   <213> Artificial Sequence
<220>
   <223> FOXL2 S33 phospho peptide
<220>
   <221> MOD_RES
   <222> (7)
   <223> PHOSPHORYLATION,
<400> 21
   Pro Glu Gly Pro Pro Pro Ser Pro Gly Lys Gly
   1               5                   10